# EUROPEAN PATENT APPLICATION

(11) **EP 2 976 985 A1**
(43) Date of publication of application: **27.01.2016**
(21) Application number: 14830491.8
(22) Date of filing: 13.06.2014
(51) Int. Cl.: A61B 1/00

(54) **CAPSULE ENDOSCOPE**

(30) Priority: 30.05.2014 JP 2014113526
(71) Applicant: MU Ltd., Shiga 520-2194 (JP)
(72) Inventor: OHTSUKA Naotake, Otsu-shi Shiga 520-0801 (JP); FUJITO Yoshiaki, Mukou-shi Kyoto 617-0002 (JP); OHTSUKA Yoshitake, Fukuoka-shi Fukuoka 810-0062 (JP)
(74) Representative: Stöckeler, Ferdinand
(86) International application number: PCT/JP2014/065744
(87) International publication number: WO 2015/181993

(57) **Abstract**

Provided is a capsule endoscope including a capsule body, a fin portion, and a sack portion, the capsule endoscope being capable of moving easily even in a situation where the movement thereof is likely to be obstructed. This capsule endoscope 1 includes: a capsule body 3 including a forward imaging element that images a forward side and a backward imaging element that images a backward side; a fin portion 4 in which a magnet that is displaced in response to an alternating magnetic field is mounted and which vibrates by being elastically deformed by the displacement of the magnet; and a sack portion 5 to which a front end 4b of the fin portion 4 is bonded at a position shifted downward from an axial line of the capsule body 3, and which is attached to the capsule body 3 in such a way that a rear end side of the capsule body 3 is inserted into the sack portion 5 and the sack portion 5 is elastically deformed to make close contact with the periphery of the rear end side, the sack portion 5 being provided with an opening of a hollow portion near the front end of the fin portion 4. The fin portion 4 moves by vibrating in an up-down direction in a liquid medium.

## Description

### [TECHNICAL FIELD]

The present invention relates to a self-propelled capsule endoscope that acquires in-vivo images.

### [BACKGROUND ART]

In recent years, a medical device that examines the inside (the digestive tract or the like) of a body using a capsule endoscope is known. The capsule endoscope in this medical device generally imposes less burden on a subject because, unlike conventional endoscopes, it does not require a tube that passes through the gullet or the like for operating the endoscope. When a subject swallows the capsule endoscope, the capsule endoscope images the surroundings with an internal imaging element to acquire images while moving through the inside of the body by a peristaltic movement of the digestive tract or the like. The capsule endoscope transmits the acquired images to an external device that controls the capsule endoscope from the outside of the body. The external device can examine the inside of the body based on the images, collect body liquids, tissues, and the like using a collector mounted on the capsule endoscope, or conduct treatments (for example, with medicines or the like) using a treatment instrument mounted on the capsule endoscope. After that, the capsule endoscope is discharged outside from the anus.

Among such capsule endoscopes, a self-propelled capsule endoscope capable of moving to a destination to be examined by itself as well as moving passively by the peristaltic movement has been proposed from the past. For example, Patent Documents 1 and 2 disclose capsule endoscopes in which a magnet having a magnetization direction in an axial direction (longitudinal direction) is mounted and a fin portion is provided. The fin portion is bonded to a rear end of a capsule body (endoscope body) in which an imaging element is included. These capsule endoscopes are configured such that the magnet vibrates in response to an alternating magnetic field generated by an external device, and the fin portion vibrates by bending with the vibration of the magnet to push surrounding liquid backward, whereby the capsule endoscope moves in the axial direction.

A method of bonding the fin portion to the capsule body includes a method of attaching the fin portion directly to the capsule body using an adhesive or the like. Moreover, Patent Document 1 disclosed a method of connecting a bag-shaped sack portion made from rubber to the fin portion and attaching the sack portion to the rear end of the capsule body. Such a sack portion enables the fin portion to be easily connected to the capsule body.

### [PRIOR ART DOCUMENTS]

### [PATENT DOCUMENTS]

Patent Document 1: Japanese Patent Application Publication No. 2008-279019
Patent Document 2: Japanese Patent Application Publication No. 2013-066694

### [SUMMARY OF THE INVENTION]

### [PROBLEM TO BE SOLVED BY THE INVENTION]

Incidentally, the capsule endoscope may encounter a situation inside the body where the movement thereof is likely to be obstructed. An example of such a situation is a case where there is a very small amount of liquid inside the digestive tract or the like and a case where the digestive tract or the like has too many folds so that the fin portion is sandwiched between the folds.

The present invention has been made in view of the foregoing, and an object thereof is to provide a capsule endoscope including a capsule body, a fin portion, and a sack portion, the capsule endoscope being capable of moving easily even in a situation where the movement thereof is likely to be obstructed.

### [MEANS FOR SOLVING THE PROBLEM]

In order to attain the object, according to an embodiment of the present invention, there is provided a capsule endoscope including: a capsule body including a forward imaging element that images at least a forward side; a fin portion in which a magnet that is displaced in response to an alternating magnetic field is mounted and which vibrates by being elastically deformed by the displacement of the magnet; and a sack portion to which a front end of the fin portion is bonded, and which is attached to the capsule body in such a way that a rear end side of the capsule body is inserted into the sack portion and the sack portion is elastically deformed to make close contact with the periphery of the rear end side, wherein the fin portion moves by vibrating in an up-down direction in a liquid medium.

Preferably, a position where the front end of the fin portion is bonded to the sack portion is shifted downward from an axial line of the capsule body.

Preferably, the capsule body further includes a backward imaging element that images a backward side, and the sack portion has an opening of a hollow portion near the front end of the fin portion.

Preferably, a through-hole is formed in a portion of the fin portion.

### [EFFECTS OF THE INVENTION]

The capsule endoscope of the present invention can easily move in a situation where the movement thereof is likely to be obstructed.

### [BRIEF DESCRIPTION OF DRAWINGS]

Fig. 1 is a schematic diagram illustrating a usage state of a capsule endoscope according to an embodiment of the present invention;
Fig. 2 is a perspective rear view of the capsule endoscope;
Fig. 3 is a perspective rear view illustrating a disassembled state of the capsule endoscope;
Fig. 4 is a side view schematically illustrating a moving state of the capsule endoscope;
Fig. 5 is a side view illustrating a schematic configuration of the capsule endoscope;
Fig. 6 is a plan view illustrating a schematic configuration of the capsule endoscope;
Fig. 7 is a perspective rear view illustrating a disassembled state of the capsule endoscope in which a through-hole is formed in a fin portion;
Fig. 8 is a perspective rear view illustrating a disassembled state of a modification of the capsule endoscope; and
Fig. 9 is a perspective rear view illustrating a disassembled state of another modification of the capsule endoscope.

### [DESCRIPTION OF EMBODIMENTS]

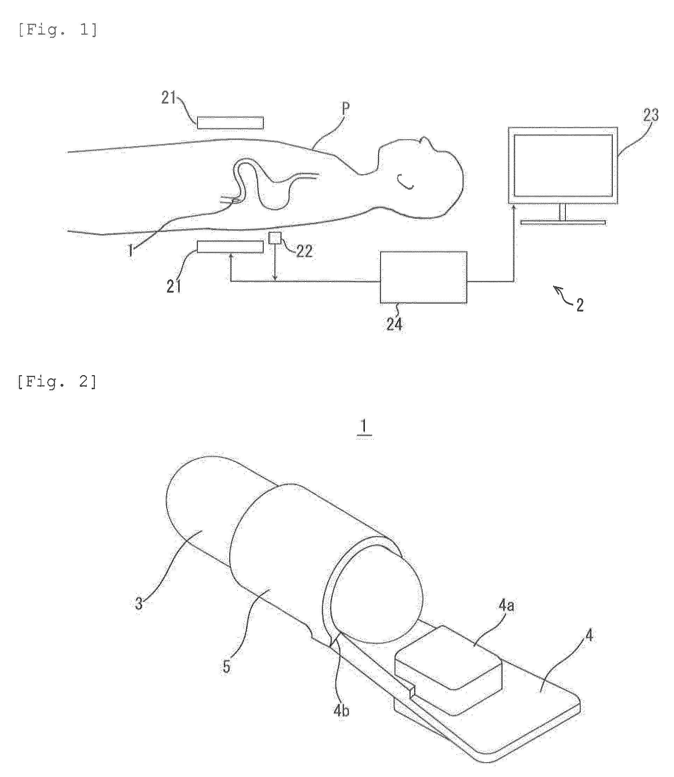

Hereinafter, embodiments of the present invention will be described with reference to the drawings. A capsule endoscope 1 according to an embodiment of the present invention performs examinations or the like of the inside (the digestive tract or the like) of the body of a subject P and is controlled by an external device 2 as illustrated in Fig. 1. The subject P is examined after swallowing the capsule endoscope 1 from the mouth. Alternatively, the subject P may be examined after the capsule endoscope 1 is inserted from the anus. The capsule endoscope 1 moves through the inside of the body of the subject P. The external device 2 may be configured to include a magnetic field generating unit 21 that generates a magnetic field, a communication unit 22 that wirelessly communicates with the capsule endoscope 1, a monitor 23, and a controller 24 that controls these components.

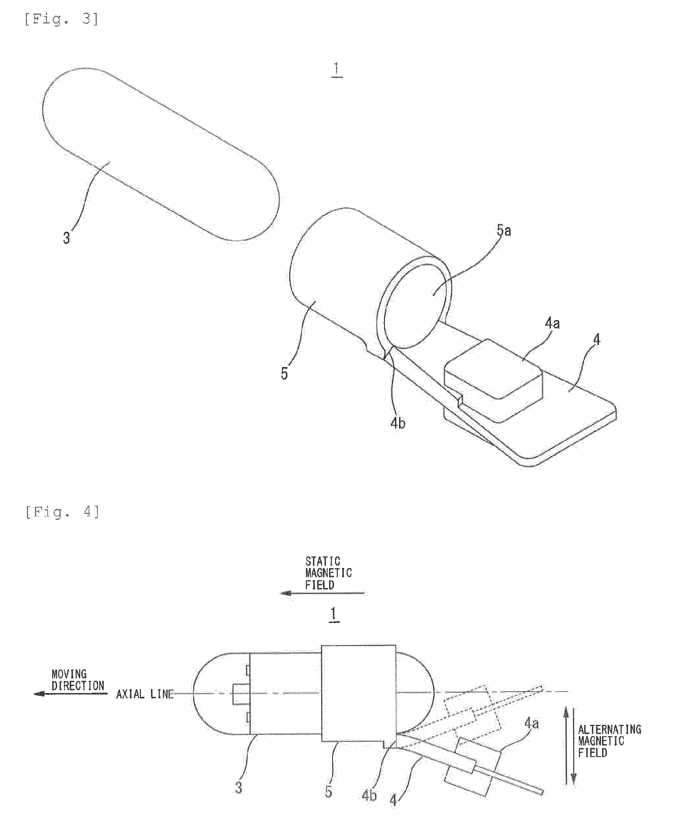

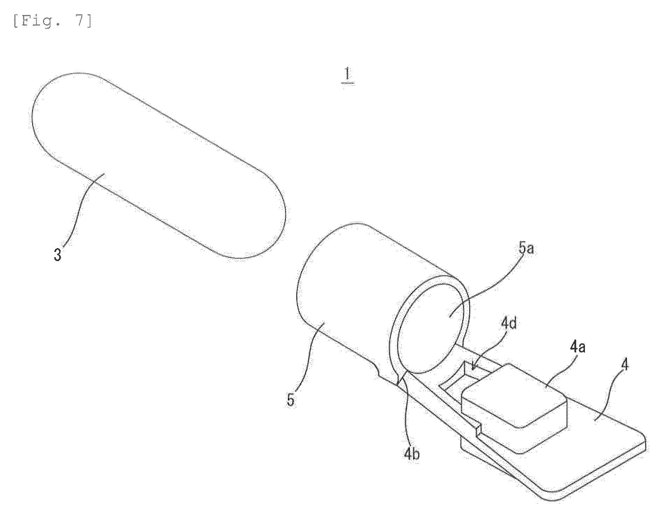

As illustrated in Figs. 2 and 3, the capsule endoscope 1 includes a capsule body 3, a fin portion 4, and a sack portion 5. In Figs. 2 and 3, members inside the capsule body 3 are not illustrated. Moreover, in Fig. 3 (and Figs. 7 to 9 described later), a hollow portion (described later) of the sack portion 5 is elastically deformed so as to be widened.

The shape and size are not particularly limited. But, in many cases, the capsule body 3 has an approximately cylindrical shape. As illustrated in Fig. 4, the capsule endoscope 1 moves toward the forward side (the left side in Fig. 4) in an axial direction (longitudinal direction) of the capsule body 3. The capsule body 3 may have a diameter (the diameter in a direction orthogonal to the axial direction) of approximately 1 cm and an axial length of approximately 2.5 cm, for example.

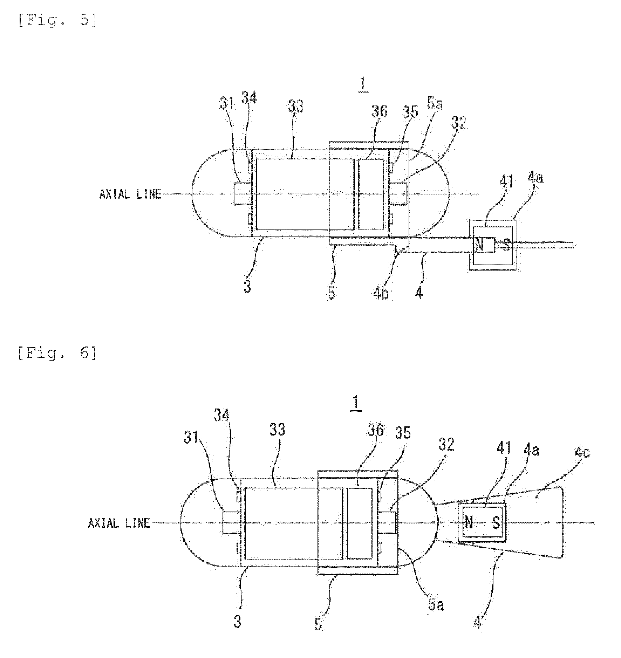

As illustrated in Figs. 5 and 6, the capsule body 3 has a forward imaging element 31 that images the forward side. A front part (the left side in Figs. 5 and 6) of a casing of the capsule body 3 is transparent so that light can pass through the front part. Moreover, the capsule body 3 preferably includes a backward imaging element 32 that images the backward side in order to check the state of the fin portion 4 described later. The forward imaging element 31 and the backward imaging element 32 may be formed of a CCD or the like.

In addition to the forward imaging element 31 or the backward imaging element 32, the capsule body 3 may include a power supply unit 33 that supplies electric power to respective units of the capsule endoscope 1, a forward illuminating unit 34 that illuminates the outside in order to allow the forward imaging element 31 to perform imaging, a backward illuminating unit 35 that illuminates the outside in order to allow the backward imaging element 32 to perform imaging, a wireless communication unit 36 that processes the images acquired by the forward imaging element 31 or the backward imaging element 32 to wirelessly transmit the images to the external device 2, and the like. The wireless communication unit 36 may be configured to wirelessly receive a control signal from the external device 2 to control the forward imaging element 31 or the backward imaging element 32, the forward illuminating unit 34 or the backward illuminating unit 35, and the like. Moreover, a collector that collects body liquids, tissues, or the like, a treatment instrument that conducts treatments (for example, with medicines or the like), and the like may be mounted on the capsule body 3. Figs. 5 and 6 schematically illustrate the respective units inside the capsule body 3 in a perspective view.

The fin portion 4 has a magnet mounting portion 4a on which a magnet 41 is mounted. Moreover, portions of the fin portion 4 other than the magnet 41 are made from a soft material such as a silicon resin. The fin portion 4 may be formed by placing the magnet 41 in a molding mold, filling a silicon resin or the like around the magnet, and hardening the silicon resin. Moreover, the fin portion 4 may have a thickness (the length in a direction orthogonal to the axial direction) of approximately 0.1 cm to 0.5 cm and an axial length of approximately 2.0 cm. Figs. 5 and 6 schematically illustrate the magnet 41 buried in the magnet mounting portion 4a of the fin portion 4 in a perspective view.

Fig. 41 has a magnetization direction in the axial direction of the capsule body 3. The magnet 41 receives a static magnetic field and an alternating magnetic field orthogonal to the static magnetic field from the external device 2. Upon receiving the static magnetic field, the magnet 41 changes its direction so that the magnetization direction is parallel to the direction of the static magnetic field. Moreover, an overall direction (the axial direction of the capsule body 3) of the capsule endoscope 1 changes according to the direction of the magnet 41.

The magnet 41 is displaced in response to the alternating magnetic field. More specifically, the magnet 41 is displaced so as to be rotated since the direction of force acting on the rear end (the S-pole side in Figs. 5 and 6) of the magnet 41 is opposite to the direction of force acting on the front end (the N-pole side in Figs. 5 and 6). In this case, since a front end 4b of the fin portion 4 is bonded to the sack portion 5 and a rear end thereof is open, which will be described later, the portion near the front end 4b is elastically deformed by the displacement of the magnet 41 in response to the alternating magnetic field, and the fin portion 4 vibrates.

More specifically, the fin portion 4 has a wide side surface 4c like the fin of a fish (see Fig. 6), and the fin portion 4 vibrates when the alternating magnetic field is incident on the wide side surface 4c in an approximately perpendicular direction (see Fig. 4). The fin portion 4 can push liquid backward efficiently with the aid of the vibrating wide side surface 4c. When the capsule endoscope 1 moves in a liquid medium, the fin portion 4 vibrates in an up-down direction (see Fig. 4) and the capsule endoscope 1 moves in the forward direction (the front side in the axial direction of the capsule body 3). Here, the up-down direction means the direction of gravity or approximately the direction of gravity (the direction away from the horizontal direction).

Here, when there is a very small amount of liquid inside the digestive tract or the like, the fin portion 4 vibrates in the up-down direction to touch and push off an inner wall of the digestive tract or the like positioned under the capsule endoscope 1, and the capsule endoscope 1 can move by the fin portion 4 receiving the counteraction of the pushing force. In order for the fin portion 4 to easily touch and push off the inner wall of the digestive tract or the like positioned under the capsule endoscope 1 and to receive the counteraction, the position where the front end 4b of the fin portion 4 is bonded to the sack portion 5 is preferably shifted downward from the axial line (central line) of the capsule body 3 as illustrated in Fig. 4 and other drawings (for example, the position is located approximately at the lower end of the capsule body 3).

The sack portion 5 is a portion that has a hollow portion and is elastically deformed easily, and, for example, may be formed from a silicon resin. The front end 4b of the fin portion 4 is bonded to the sack portion 5. The fin portion 4 and the sack portion 5 may be attached and bonded as separate members or may be integrally formed and bonded as a single member (for example, a single member made from a silicon resin). Moreover, the sack portion 5 is attached to the capsule body 3 in such a way that a rear end side of the capsule body 3 is inserted into the hollow portion of the sack portion 5 and the sack portion 5 is elastically deformed so as to make close contact with the periphery of the rear end side of the capsule body 3. The sack portion 5 allows the capsule body 3 and the fin portion 4 to be easily connected with the sack portion 5 interposed.

In the sack portion 5, an opening 5a (see Fig. 3 and other drawings) of the hollow portion is preferably formed near the front end 4b of the fin portion 4. The entire portion or a portion of the rear end portion of the capsule body 3 is exposed from the opening 5a. At least the exposed portion of the casing of the capsule body 3 is transparent so that light can pass through the portion. The state of the fin portion 4 can be checked through the opening 5a with the aid of the backward imaging element 32 of the capsule body 3. If the fin portion 4 is sandwiched between the folds of the digestive tract or the like and the capsule endoscope 1 cannot move easily, the state of the fin portion 4 may be checked and the subject may drink water or the like to accelerate the peristaltic movement of the digestive tract or the like, so that the capsule endoscope 1 can move out of the folds and move in an intended direction. The wall surface of the digestive tract or the like on the backward side may be examined through the opening 5a of the sack portion 5 with the aid of the backward imaging element 32 of the capsule body 3.

Moreover, as illustrated in Fig. 7, a through-hole 4d may be formed in a portion of the fin portion 4. By doing so, a larger area of the fin portion 4 and the wall surface of the digestive tract or the like on the backward side can be checked by the imaging element 32. Thus, in more detail, the state of the fin portion 4 can be checked and the wall surface of the digestive tract or the like on the backward side can be examined.

While the capsule endoscope according to the embodiment of the present invention has been described, the present invention can be changed in design in various ways within the scope described in the claims without being limited to those described in the embodiment. For example, a float may be added to a position near the upper end of the capsule body 3, a weight may be added to a position near the lower end of the capsule body 3, and other members may be appropriately added.

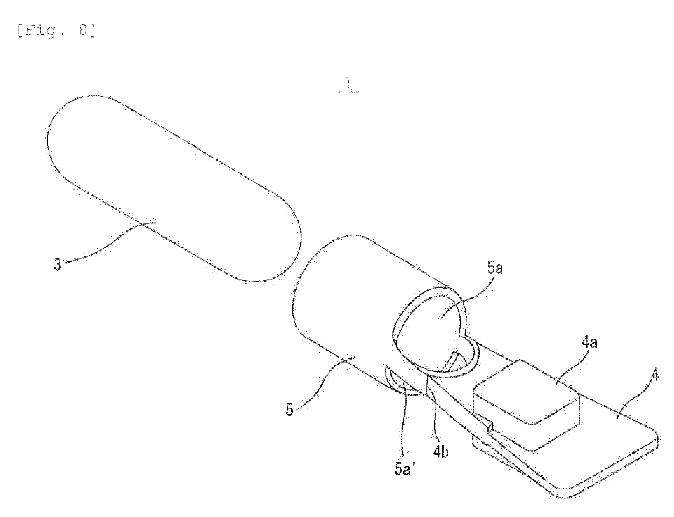

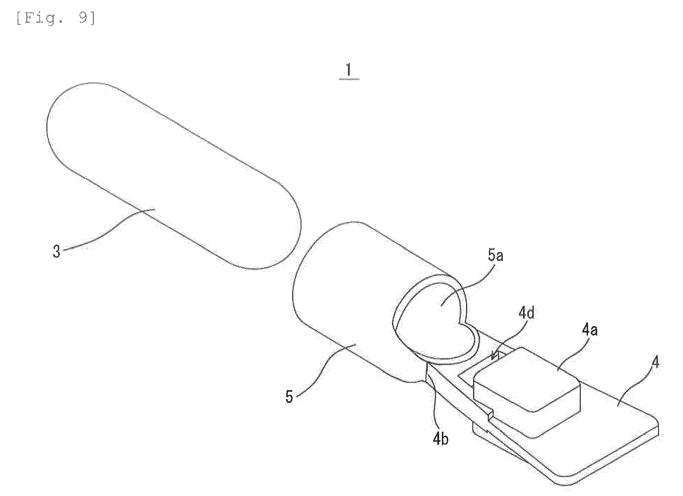

Moreover, the position where the front end 4b of the fin portion 4 is bonded to the sack portion 5 may not be shifted from the axial line of the capsule body 3 although it may become rather difficult for the fin portion 4 to touch and push off the inner wall of the digestive tract or the like positioned under the capsule endoscope 1 to receive the counteraction when there is a very small amount of liquid inside the digestive tract or the like. In this case, as illustrated in Fig. 8, openings 5a and 5a' of the hollow portion of the sack portion 5 may be formed on upper and lower sides of the front end 4b of the fin portion 4. Moreover, as illustrated in Fig. 9, the opening 5a of the hollow portion of the sack portion 5 may be formed on only one of the upper and lower sides of the front end 4b of the fin portion 4. In this case, the through-hole 4d may also be formed similarly to that illustrated in Fig. 7.

Moreover, in some cases, the feature of checking the state of the fin portion 4 may be omitted, and the backward imaging element 32 of the capsule body 3 and the opening 5a (and 5a') of the hollow portion of the sack portion 5 may be omitted.

### [EXPLANATIONS OF REFERENCE NUMERALS]

- 1:: CAPSULE ENDOSCOPE
- 2:: EXTERNAL DEVICE
- 21:: MAGNETIC FIELD GENERATING UNIT
- 22:: COMMUNICATION UNIT
- 23:: MONITOR
- 24:: CONTROLLER
- 3:: CAPSULE BODY
- 31:: FORWARD IMAGING ELEMENT
- 32:: BACKWARD IMAGING ELEMENT
- 33:: POWER SUPPLY UNIT
- 34:: FORWARD ILLUMINATING UNIT
- 35:: BACKWARD ILLUMINATING UNIT
- 36:: WIRELESS COMMUNICATION UNIT
- 4:: FIN PORTION
- 4a:: MAGNET MOUNTING PORTION
- 4b:: FRONT END OF FIN PORTION
- 4c:: SIDE SURFACE OF FIN PORTION
- 4d:: THROUGH-HOLE
- 41:: MAGNET
- 5:: SACK PORTION
- 5a:: OPENING OF HOLLOW PORTION OF SACK PORTION
- P:: SUBJECT

## Claims

1. A capsule endoscope comprising:
a capsule body including a forward imaging element that images at least a forward side;
a fin portion in which a magnet that is displaced in response to an alternating magnetic field is mounted and which vibrates by being elastically deformed by the displacement of the magnet; and
a sack portion to which a front end of the fin portion is bonded, and which is attached to the capsule body in such a way that a rear end side of the capsule body is inserted into the sack portion and the sack portion is elastically deformed to make close contact with the periphery of the rear end side, wherein
the fin portion moves by vibrating in an up-down direction in a liquid medium.

2. The capsule endoscope according to claim 1, wherein
a position where the front end of the fin portion is bonded to the sack portion is shifted downward from an axial line of the capsule body.

3. The capsule endoscope according to claim 1 or 2, wherein
the capsule body further includes a backward imaging element that images a backward side, and
the sack portion has an opening of a hollow portion near the front end of the fin portion.

4. The capsule endoscope according to claim 3, wherein a through-hole is formed in a portion of the fin portion.
